# EUROPEAN PATENT APPLICATION

(11) **EP 4 129 347 A1**
(43) Date of publication of application: **08.02.2023**
(21) Application number: 21190224.2
(22) Date of filing: 06.08.2021
(51) Int. Cl.: A61L 2/10

(54) **DISINFECTION SYSTEM USING UV LIGHT**

(71) Applicant: Koninklijke Philips N.V., 5656 AG Eindhoven (NL)
(72) Inventor: Salters, Bart Andre, 5656 AE Eindhoven (NL); Hietbrink, Roelant Boudewijn, 5656 AE Eindhoven (NL); Niessen, Eduard Matheus Johannes, 5656 AE Eindhoven (NL); Martens, Peter, 5656 AE Eindhoven (NL)
(74) Representative: Philips Intellectual Property & Standards

(57) **Abstract**

A system is provided for disinfecting an actuation surface which is for contact by a user to implement an actuation. The system comprises a light guide, a light source arrangement for delivering UV disinfection light and detection light into the light guide at a light input surface of the light guide and a detector for detecting light at a light detection surface of the light guide, for detecting at least touching of the actuation surface. The light source arrangement faces the detector along a primary optical path but the intensity of the light output in the direction of the detector is reduced. This improves the sensitivity for detecting a touch event.

## Description

### FIELD OF THE INVENTION

This invention relates to a system for disinfecting surfaces, in particular actuation surfaces, by which is meant surfaces which are contacted by hand by a person to implement a function of a device or object.

### BACKGROUND OF THE INVENTION

There are numerous objects in daily life which are meant to be used as an actuator to be manipulated by hand, to achieve a certain goal. Examples are control buttons (for example for an elevator), door knobs or levers, toilet seats, touchscreens, ATM machines, payment terminals, vending machines, as well as more common tools such as hammers and screwdrivers.

In many cases, these actuators are used in public spaces, and as a consequence, they might be used and hence touched by numerous people in a short span of time. This provides an excellent way of spreading diseases, if contagious people handle such surfaces.

It is highly desirable to come up with a method of disinfecting such surfaces, especially those in public spaces.

Lighting systems for application to a surface to provide disinfection at the surface, for example to prevent biofouling, are known. In particular, it is well-known that most micro-organisms are killed, rendered inactive or unable to reproduce with sufficient UV radiation. This effect is mainly governed by the total dose of UV radiation. A typical dose to kill 90% of a certain micro-organism is 10m Wh per square meter. Disinfection involves the killing of a fraction or all of a range of target micro-organisms.

Ultraviolet (UV) is that part of electromagnetic light bounded by the lower wavelength extreme of the visible spectrum and the X-ray radiation band. The spectral range of UV radiation is by definition between 100 and 400 nm and is invisible to human eyes. Using the CIE classification the UV spectrum is subdivided into three bands:
UVA (long-wave) from 315 to 400 nm
UVB (medium- wave) from 280 to 315 nm
UVC (short-wave) from 100 to 280 nm

Various light sources for generating UV are known, such as low-pressure mercury discharge lamps, medium pressure mercury discharge lamps and dielectric barrier discharge lamps.

A preferred option is low cost, low power UV LEDs. LEDs can generally be included in smaller packages and consume less power than other types of light sources. LEDs can be manufactured to emit (UV) light of various desired wavelengths and their operating parameters, most notably the output power, can be controlled to a high degree. A suitable germicidal dose can easily be achieved with existing UV LEDs, for example UVC LEDs.

WO 2018/215272 discloses a system for disinfecting a surface of a light guide by providing UV radiation into the light guide, wherein UV radiation exits the light guide to provide a disinfecting function to the surface of the light guide. An amount of light is detected which is dependent on the amount of UV radiation which is internally reflected (i.e. which did not exit the light guide and was not absorbed) and this can be used to determine if the surface has been contacted. The UV radiation may then be reduced or switched off when it is detected that a person is touching the surface, and hence they are close to the surface, to reduce UV exposure of the person.

WO 2018/215272 relates primarily to the prevention of biofouling of surfaces which are maintained under water, such as the hull of a ship. However, an example given in WO 2018/215272 is a door knob in which UV radiation is used to keep the knob clean, but the radiation is switched off when the door knob is contacted.

It would be desirable to improve the behavior of the known systems, in particular to increase the sensitivity of the detection of human touch, so that the control of UV generation is more reliably performed.

### SUMMARY OF THE INVENTION

The invention is defined by the claims.

According to examples in accordance with the invention, there is provided a system for disinfecting an actuation surface which is for contact by a user to implement an actuation, the system comprising:
a light guide;
a light source arrangement for providing UV disinfection light and detection light in the light guide; and
a detector for detecting the detection light at a light detection surface of the light guide, wherein a primary optical path is provided between the light source arrangement and the detector,
wherein the light source arrangement adapted to deliver detection light with an intensity of the detection light output along the primary optical path less than an intensity of at least one output angularly offset from the primary optical path.

The invention provides a disinfecting system for disinfecting a surface to be touched by a user. The detecting of touching can be used to ensure that the delivery of UV disinfection light is provided at suitable times, for example to ensure disinfection after a touch event and/or to reduce or turn off the generation of disinfection light during touching. Thus, controlling the light source arrangement may involve turning on or off the disinfection light of the light source arrangement, or more generally it may involve controlling the intensity e.g. between full intensity and zero intensity or to intermediate intensity levels.

The primary optical path is a main path from the light source arrangement to the detector. It may be a direct path, so that the light source arrangement faces the detector, but there may be one or more reflections along the primary path. Thus, in a line of sight system, the light source arrangement faces the detector, but in an alternative design, the light source arrangement does not need to directly face the detector. The primary path for example does not include any interaction with the surface at which a touch event is to be detected. It may for example include reflection by an internal reflector for example to enable the system to be designed with a desired shape. The primary optical path is for example the shortest optical path between the light source and the detector. The aim is to reduce the light intensity along this shortest optical path.

The light source arrangement is for example external to the light guide and delivers the UV disinfection light and the detection light to a light input surface of the light guide. However, the light source arrangement may instead be integrated into the light guide.

The primary path is thus not aligned with the direction of peak intensity. The angularly offset light may be considered to define a set of secondary paths. In a rotationally symmetrical system, the primary path may be at a local minima of the light intensity, so that all around the direction of the primary path, there is an initial increase in intensity (i.e. a positive gradient of intensity in all directions outwardly from the primary path direction). However, this is not essential and instead at least one of these secondary paths has a higher intensity than the primary optical path.

The detector for example comprises a light detector for detecting a light level inside the light guide, and the controller is adapted to detect touching by detecting a change in the detected light level. For example, the touching alters the refractive index boundary conditions and thus allows more light to escape from the light guide. In other scenarios, the detector may observe an increase, for example if a highly reflective object contacts the light guide.

The invention makes use of particular optical output characteristics from the light source arrangement (for example by means of a beam shaping arrangement) to reduce the path of light along a primary path between the light source arrangement and the detector. This light is not influenced by a touch event and it thereby provides a baseline signal to the detector which can drown out the changes in light level to be detected. Thus, by reducing this direct light path, the sensitivity of the detection is improved.

The system is for example configured such that an intensity of the light output along the primary optical path is less than an intensity of the light output in at least one direction angularly offset by 10 degrees from the primary optical path. Thus, the light output intensity along the primary optical path is a local minima in the light output intensity.

The system may be configured such that an intensity of the light output along the primary optical path is below 75%, preferably below 50%, of a maximum intensity. The maximum intensity is thus offset from the direction of primary optical path, i.e. towards the the detector, and the intensity in this direction is significantly lower than the maximum intensity.

The system may be configured such that an intensity of the light output has a peak intensity in a direction angularly offset from the primary optical path in a range 20 to 45 degrees. The peak intensity is nevertheless at an angle for which the conditions for total internal reflection are met, i.e. below the critical angle. For typical light guide materials (such as plastics and glass), the refractive index at UV wavelengths is for example in the range 1.3 to 1.6 corresponding to critical angles in the range 42 to 50 degrees (assuming an interface to air). This corresponds to a maximum light emission direction, relative to the primary path (e.g. parallel to the surface of the light guide), of 40 to 48 degrees. Thus, the peak intensity is below the maximum light emission angle for meeting the critical angle, so that most of the light will be totally internally reflected (depending on the exact angular distribution of the light).

The system may be configured such that an intensity of the light output has a peak intensity in a direction (within the light guide) angularly offset from the primary optical path in by less than 48 degrees, such as in the range 30 to 45 degrees. This means light is steered away from the primary optical path as far as possible while remaining within the range for total internal reflection.

The system for example provides a batwing intensity profile. Beam shaping arrangements, i.e. lenses, for creating a batwing intensity profile from a Lambertian LED output are well known.

The light guide for example has a thickness in a width direction perpendicular to the primary optical path, and the light guide has a length of the primary optical path which preferably is at least 10 times the thickness, for example at least 20 times the thickness, for example at least 30 times the thickness.

By creating a light guide which is long (in the direction towards the detector) relative to the perpendicular width, it can be ensured that there are multiple reflections of the light offset from the direct path before the light reaches the detector. Thus, there are more interactions with the surface, so that more of the light paths will be interrupted by the presence of a contact (e.g. finger). This again improves the sensitivity.

The light guide may comprise an annular cylinder. This provides one way to implement a thin width compared to the length.

The light source arrangement may comprise a UV light source for generating both the disinfection light and the detection light as a same light path.

The UV light source for example comprises a UVC LED. UVC LEDs are now widely available with the required electrical and optical performance.

The system preferably further comprises a controller for identifying touching of the actuation surface based on the detected detection light and for controlling the light source in response to said touching. The controller may be sold as a separate part to the remainder of the system.

The controller is preferably adapted to detect touching of the actuation surface by detecting a change in the detected light level and is adapted to control the light source arrangement in dependence on the detected touching. The controller is for example adapted to (immediately) turn off the light source arrangement (in particular the generation of the UV disinfection light) during detected touching. This reduces the exposure of a person to the generated UV radiation.

The UV disinfection light may then be turned on after the detected touching has ended to provide a disinfecting function.

The controller may be further adapted to detect contamination of the actuation surface by detecting a change in light out-coupled from the light guide (a small change, less than that when the surface is touched), and retain the UV disinfection light turned on in response to detected contamination. In this way, the light source arrangement remains activated if out-coupling is changed (e.g. increased) due to surface contamination.

The controller may be adapted to analyze temporal characteristics of the light detected by the detector to distinguish between touching events and said contamination. The rate of change over time is a strong indicator of human touch, versus contamination which builds up more slowly over time. Thus, the detection may be based both on the absolute value of a detected signal as well as the temporal characteristics.

The controller may be further adapted to detect a level of wear and tear of the light guide. For example, scratches may be detected as they also cause an increase in light out-coupling and again with different temporal characteristics to a hand touching event or contamination.

The light guide is for example for application over a base, which is the surface to which actuation is provided through the disinfecting system.

In one example, the base is a door handle, and the light guide comprises a tube for sliding over the base to define an outer handle surface. Thus, the system can provide disinfection of door handles.

In another example, the base is a touch or push button, and the light guide comprise a patch for application over the base to define an outer button surface. Thus, the system can provide disinfection of individual buttons.

In another example, the base is a touch or push keypad, and the light guide comprises a patch for application over the base to define an outer keypad surface. Thus, the system can provide disinfection of a keypad.

In another example, the base is a handle of a tool and the light guide comprises a sleeve for application over the handle.

The invention also provides a manually operated device, comprising:
a base; and
the system as defined above defining an actuation surface for transferring actuation to the base through the light guide,
wherein the actuation of the base is for implementing operation of the device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known arrangement of a light guide and a UV light source for disinfecting a surface;
Fig. 2 shows a batwing intensity profile;
Fig. 3 is used to explain the conditions for total internal reflection;
Fig. 4 shows a light guide with typical relative dimensions;
Fig. 5 shows a system in accordance with an example of the invention; and
Fig. 6 shows a light guide in the form of an annular cylinder.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for disinfecting an actuation surface which is for contact by a user to implement an actuation. The system comprises a light guide, a light source arrangement source for delivering UV disinfection light and detection light into the light guide at a light input surface of the light guide, and a detector for detecting light at a light detection surface of the light guide, for detecting at least touching of the actuation surface. The light source arrangement faces the UV detector along a primary optical path but the intensity of the light output in the direction of the detector is reduced. This improves the sensitivity for detecting a touch event.

The invention relates generally to any manually operated device. Such devices have in common a surface which is to be handled by a user, wherein a force or just contact with the surface is for implementing operation of the device.

For example, in the case of a door handle, a force to the surface causes the handle to rotate about a pivot axis and thereby operate a door latch. The same general function is implemented by any manual actuator. For example, for a touch or push button (e.g. in a lift or an ATM machine) there is a surface which is the touch or push button. For a touch or push keypad the surface is an array of buttons. A force to the button or buttons causes the button or buttons to move (depress) or simply register and thereby actuate a corresponding circuit.

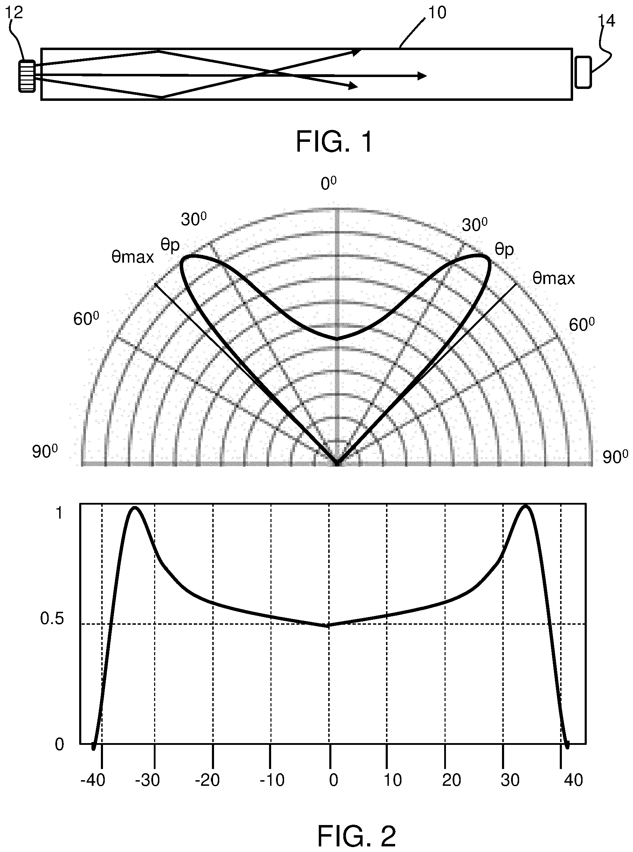

Fig. 1 shows the known arrangement of a light guide 10 having a light source arrangement such as one or more UVC LEDs 12 at one end and a detector 14 at an opposite end. The light guide 10 may for example be used to cover a surface or button or any other actuated surface.

The light guide is for example a glass, quartz or plastics layer. Any suitable material may be used which is transparent to the disinfection light to be used. The refractive index of the light guide creates a refractive index difference with respect to air at the outer surface to implement the required total internal reflection to guide the light inside the material. The resulting external contact surface becomes the actuation surface of the overall device.

The UVC LED light source 12 emits light into the light guide. For example, the UVC LED may have a Lambertian light output intensity profile. The light is captured within the light guide by total internal reflection. Output light is however coupled out from the light guide in areas where there is any form of contamination (i.e. virus or bacteria particles). These interrupt the total internal refection by means of the change in refractive index (compared to air). This provides the desired disinfection function. However, light will also be coupled out when a finger or hand touches the surface.

This touch event will cause a larger amount of light to be coupled out, which can be detected easily and this may be used to interrupt delivery of the output light when there is a touch event, to decrease exposure to the UVC light. It is desirable to detect even small touch events (e.g. a single finger, or a light touch), to further decrease the potential exposure to UVC light.

The amount of out-coupling of light thus depends at least in part on the surface properties of the light guide. In particular, light will escape if the conditions for total internal reflection are not met.

There may be some deliberate out coupling of light even without contamination or detection of touch. This may for example be achieved by incorporating scattering particles in the light guide so that some of the light will be scattered to have an angle that allows out-coupling. Alternatively, a surface pattern may be used which defines light out-coupling features. The design of the light out-coupling features can be optimized to ensure a uniform out-coupling of light from all of the actuation surface or desired contact areas of the actuation surface. This is a more direct way of coupling light out from the light guide.

The design may be such that there is very little, if any, out-coupling in the case of a clean surface. The light may escape when and where there is biological contamination present on the surface. A small amount of base radiation escaping from the light guide however provides a tolerance margin.

In the example shown, the detection of the UV light which has not escaped provide a way to determine how much UV light has escaped. Thus, the UV light functions as both disinfection light and also as detection light. As explained below, the disinfection and touch detection functions may however use different light.

The invention provides an arrangement which increases the sensitivity of the detection of a touch event.

As shown in Fig. 1, some of the light delivered by the UVC LED 12 will travel straight forward, and reach the detector 14 uninterrupted, regardless of the condition at the surface of the light guide. This straight forward path is a primary optical path. The primary optical path does not however need to be a straight line of sight path. It may include one or more reflections.

However, some other rays of light will be emitted at an angle, and bounce inside the light guide, due to total internal reflection. If an object, such as a finger or hand, touches the surface, it will disturb this total internal reflection for those angled light emissions. Thus, less light will reach the detector 14, and this drop in detected signal intensity is used as the detection of a touch event.

An issue is that the light that travels in a more or less straight line (or along the primary optical path, more generally) gives a strong signal on the detector; and as a result, registering a touch event is not easy as only a small reduction in detected intensity results. For example, the intensity on the detector might be 100 (arbitrary) units during a non-touch situation; and may drop no lower than 90 units after a touch event, because there is no influence on the light travelling straight. The ratio of direct versus indirect light is thus not ideal.

The invention provides two measures to address this issue.

The first measure is to provide beam shaping of the output of the UVC LED 12, in particular in order to reduce the forward component of the light.

This may be achieved by having a suitably designed LED chip or by adding a beam shaping arrangement to a LED chip which does not have the desired light output characteristics. A beam shaping arrangement is for example used for shaping the (detection) light output from the light source arrangement such that an intensity of the light output in the direction of the detector is reduced. In particular, an intensity in the direction of the primary optical path (i.e. normally to the substrate of the LED and directly towards the detector in the example shown) is reduced to be less than an intensity of the light output angularly offset from the direction of the primary optical path (i.e. towards the detector in the example shown). In this way, the intensity in the forward direction is a local minima of the output intensity distribution.

This may be achieved using beam shaping optics such as a lens over a LED chip, or else optics may be incorporated into a LED package itself. Thus, off-the-shelf LED packages may be used with no modification needed.

One example of suitable intensity profile is a batwing intensity profile as shown in Fig. 2.

The top image show the so-called batwing profile as an angular intensity plot. The angle zero corresponds to a forward emission direction, normally away from the LED substrate towards the detector. A peak intensity no longer arises in this forward direction, but the peak is shifted to an angle 0p. Fig. 2 shows a peak at around 35 degrees. The light output is substantially all within a maximum angle θmax. This is below the angle corresponding to the critical angle so that substantially all the light is confined with the light guide.

A critical angle of 42 degrees corresponds to a refractive index interface between a material of refractive index of 1.5 and air. The angle to the primary path would then be 48 degrees. Thus θmax (inside the light guide material) may for example be below 48 degrees. It is noted, however, that assuming the LED output with the batwing profile is in air, and is emitted towards and into the light guide, light will be refracted at the refractive index boundary into the light guide, resulting in light having smaller angles inside the light guide than outside (when just exiting the LED). Thus, depending on the refractive index of the light guide, angles up to 60 to 70 degrees in the LED output profile may still be captured within the total internal reflection angle.

The bottom image shows a normalized intensity plot versus angle.

The beam shaping arrangement is configured such that an intensity of the light output in the direction of the detector (0 degrees in Fig. 2) is less than an intensity of the light output which is angularly offset, e.g. by 10 degrees, from the primary optical path. Thus, there is a local minima of the intensity in the forward direction as can be seen in the lower image of Fig. 2.

An intensity of the light output in the direction of the detector (0 degrees in Fig. 2) is preferably below 75% of the maximum intensity (the intensity at θp) and preferably even below 50%. Fig. 2 shows an example where the forward intensity is 50% (normalized value 0.5) of the maximum intensity (normalized value 1).

The intensity of the light output for example has a peak intensity in a direction angularly offset from the primary optical path by the angle θp in a range 20 to 45 degrees, such as 30 to 45 degrees. Thus, as much of the detection light as possible is directed away from the detector (by shifting the peak intensity away from the forward direction) but still ensuring the conditions for total internal reflection are met.

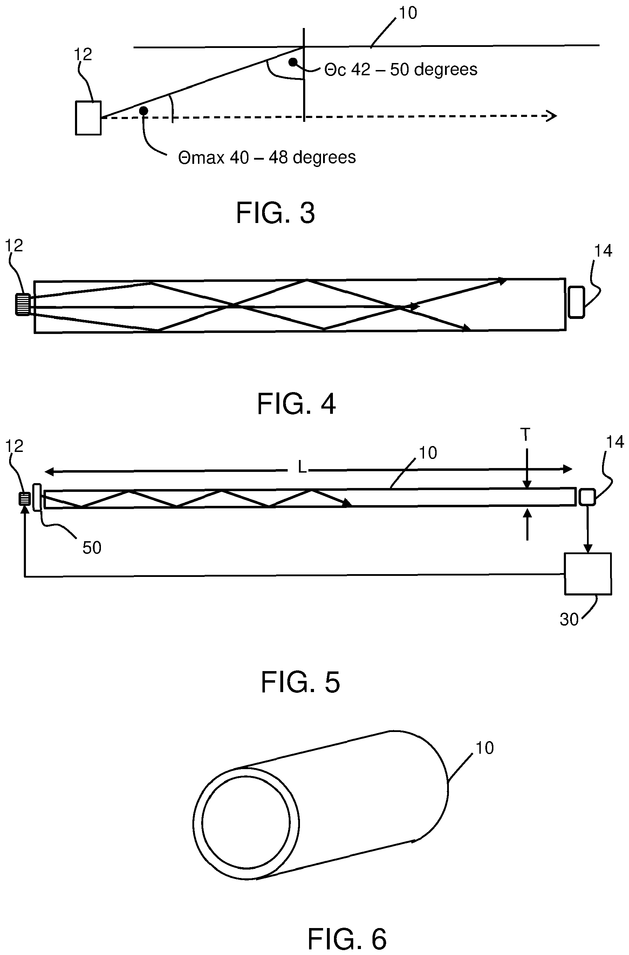

Fig. 3 explains these conditions. The critical angle θc depends on the refractive index of the light guide material. For plastics and glass, it is typically in the range 1.3 to 1.5 at the UV wavelengths of interest. For example, PMMA has a refractive index of approximately 1.5 (depending on the particular wavelength).

The range of refractive indices 1.3 to 1.5 corresponds to a critical angle in the range of 42 to 50 degrees. In turn, this means the maximum angle of offset θmax from the direct path to the detector within the light guide material is in the range of? 40 to 48 degrees to ensure all output light satisfies the conditions for total internal reflection. The intensity of the light output, once within the light guide material, for example has a peak intensity in a direction angularly offset from the primary optical path below and below the maximum angle of offset corresponding to the particular refractive index values (and hence below 48 degrees), for example in a range 30 to 45 degrees

A second measure is to provide a thin light guide. In particular, the ratio of the thickness versus the length is reduced. This ensures that light bounces more often (has more interactions with the surface), which increases the chance that a human finger or hand disturbs the path of the light.

Fig. 4 shows a light guide with typical relative dimensions.

Fig. 5 shows a thin light guide. It also shows a beam shaping component 50 as described above although this may be an integral part of the LED package as mentioned above. Fig. 5 also shows the controller 30 for controlling the light source in dependence on the detected light.

The light guide for example has a thickness T in a width direction perpendicular to the direction between the light source arrangement 12 and the detector 14. The light guide has length L between the light source and the detector which is at least 10 times the thickness T, for example at least 20 times the thickness, for example at least 30 times the thickness.

For example, the door handle may be around 10cm long, with a 0.5cm thickness sleeve, or an elevator button may have a 3cm diameter with a 1.5mm thick cover.

This thickness is for example the direction of lowest dimension. The light guide for example comprises a 2D area to cover a surface to be sterilized, and the thickness direction is perpendicular to the plane of the surface. Thus, an actuation force is applied to an underlying actuation surface through the light guide in the thickness direction.

Fig. 6 shows that the light guide may achieve the desired small thickness by comprising an annular cylinder which surrounds a non-planar surface to be sterilized, such as a door handle. A thin light guide may instead be formed by applying a sheet of transparent material (e.g. silicone) on top of a core such as a steel cylinder for strength and support.

As mentioned above, a controller may turn off the light source during detected touching. This reduces the exposure of a person to the generated UV radiation. The light source may then be turned on after the detected touching has ended to resume the disinfecting function. The controller may also detect contamination of the actuation surface by detecting an increase in light out-coupled from the light guide, but by an amount less than when the surface is touched. The light source may remain turned on in response to detected contamination. In this way, the light source remains activated if out-coupling is increased due to surface contamination.

The controller may also detect a level of wear and tear of the light guide. For example, scratches may be detected as they also cause an increase in light out-coupling. Thus, both the level of light out-coupling (and hence the corresponding change in the detected light) and the temporal characteristics may be used to identify different surface conditions of the exposed surface.

As an optional additional feature, the controller can provide an output to indicate when the system should be replaced. The detector may also be used as a safety sensor to ensure the UV radiation is not too high e.g. as a result of a component failure of the light source. If a safety risk is possible (especially when the exact cause is unknown), a decision to turn off the light may then be appropriate.

The detection of touching by monitoring the light level requires the light source to be initially turned on. The light source may be turned on permanently (apart from during detected touch events) so that the detection function is always active.

When the light source is turned off, detection based on light detection is no longer possible. For this purpose, it is necessary to use an alternative way to determine when exactly the touch event has ended.

One option is to probe the light source with very short (∼ms) pulses of UV light to allow intermittent detection to take place, or a visible light source (which is not harmful) may be used for the detection when the UV light source is deactivated. Such a visible light source may also be pulsed so that it is not visible to the human eye.

Indeed, the whole detection may be based fully on the use of visible light or even infrared light (giving reduced sensitivity to environmental light).

Various other algorithms could be applied for the on/off decision for example based on the trade-off between power saving and disinfection rate (since e.g. 10mWh will provide for 90% disinfection, and 20mWh thus provides for 99%, etc.). A quick, short, high power disinfection right after a touch event might even be followed by a low power, permanent, maintenance dose.

The beam shaping may provide a rotationally symmetric intensity pattern about the forward direction. However, an asymmetric intensity profile is also possible. For example, for a light guide covering a 2D surface, an array of light sources and detectors may be used. The light should spread between the light sources to cover the full area of the surface, but a path should also be maintained to the detectors so that the amount of out coupling can be detected.

Thus, the beam shaping function will be designed taking into account the required surface illumination as well as detection function.

The examples above make use of a UV light source such as a UV LED for providing disinfection light and the same light is used for detection. However, as also mentioned above, the touch detection may use different light. Thus, more generally, there is a light source arrangement which provides UV disinfection light and detection light. This may be the same light from a single light source, or there may be multiple light sources in a light module providing different wavelengths of light, one for disinfection and one for detection.

The invention is of particular interest for devices used and shared in public spaces, such as a public door knob (e.g. of a restaurant or public toilet etc.).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A system for disinfecting an actuation surface which is for contact by a user to implement an actuation, the system comprising:
a light guide (10);
a light source arrangement (12) for providing UV disinfection light and detection light in the light guide; and
a detector (14) for detecting the detection light at a light detection surface of the light guide, wherein a primary optical path is provided between the light source arrangement and the detector,
wherein the light source arrangement adapted to deliver detection light with an intensity of the detection light output along the primary optical path less than an intensity of at least one output angularly offset from the primary optical path.

2. The system of claim 1, wherein the light source arrangement is adapted such that an intensity of the detection light output along the primary optical path is less than an intensity of the light output angularly offset by 10 degrees from the primary optical path.

3. The system of claim 1 or 2, wherein the light source arrangement is adapted such that an intensity of the detection light output along the primary optical path is below 75%, preferably below 50%, of a maximum intensity.

4. The system of any one of claims 1 to 3, wherein the light source arrangement is adapted such that an intensity of the detection light output has a peak intensity in a direction angularly offset from the primary optical path below 48 degrees, for example in the range 30 to 45 degrees.

5. The system of any one of claims 1 to 4, wherein the light source arrangement is adapted to provide a batwing intensity profile.

6. The system of any one of claims 1 to 5, wherein light guide (10) has a thickness (T) in a width direction perpendicular to the direction of the primary optical path, wherein the light guide has length (L) along the primary optical path which is at least 10 times the thickness, for example at least 20 times the thickness, for example at least 30 times the thickness.

7. The system of any one of claims 1 to 6, wherein the light guide (10) comprises an annular cylinder.

8. The system of any one of claims 1 to 7, wherein the light source arrangement comprises a UV light source (12) for generating both the disinfection light and the detection light as a same light path.

9. The system of claim 8, wherein the light source arrangement comprises a UV LED.

10. The system of any one of claims 1 to 9, further comprising a controller (30) for identifying touching of the actuation surface based on the detected detection light and for controlling the light source in response to said touching,

11. The system of claim 10, wherein the controller is adapted:
to detect touching of the actuation surface by detecting a change in the detected light level; and optionally
to turn off the UV disinfection light during detected touching.

12. The system of claim 11, wherein the controller (30) is further adapted to detect contamination of the actuation surface by detecting a change in light out-coupled from the light guide, and retain the UV disinfection light turned on in response to detected contamination.

13. The system of any one of claims claims 10 to 12, wherein the controller (30) is adapted to analyze temporal characteristics of the light detected by the detector.

14. The system of any one of claims 1 to 13, wherein the light guide (10) is for application over a base, wherein:
the base is a door handle, and light guide comprise a tube for sliding over the base to define an outer handle surface; or
the base is a touch or push button, and the light guide comprises a patch for application over the base to define an outer button surface; or
the base is a touch or push keypad, and the light guide comprises a patch for application over the base to define an outer keypad surface; or
the base is a handle of a tool and the light guide comprises a sleeve for application over the handle.

15. A manually operated device, comprising:
a base; and
the system of any one of claims 1 to 14 defining an actuation surface for transferring actuation to the base through the light guide,
wherein the actuation of the base is for implementing operation of the device.
